# EUROPEAN PATENT APPLICATION

(11) **EP 4 356 873 A1**
(43) Date of publication of application: **24.04.2024**
(21) Application number: 22824892.8
(22) Date of filing: 08.06.2022
(51) Int. Cl.: A61C 19/06, A61N 1/18

(54) **PERI-IMPLANTITIS TREATMENT DEVICE AND METHOD FOR ADJUSTING BIOFILM ACTIVITY**

(30) Priority: 16.06.2021 JP 2021099813
(71) Applicant: National Institute for Materials Science, Tsukuba-shi, Ibaraki 305-0047 (JP)
(72) Inventor: OKAMOTO Akihiro, Tsukuba-shi, Ibaraki 305-0047 (JP); MIRAN Waheed, Tsukuba-shi, Ibaraki 305-0047 (JP)
(74) Representative: J A Kemp LLP
(86) International application number: PCT/JP2022/023140
(87) International publication number: WO 2022/264901

(57) **Abstract**

A peri-implantitis treatment device (1) according to the present invention includes: an electrode (14) for applying a negative electric potential to a metal portion (7) of a dental implant (8); a potential controller for controlling the electric potential of the electrode (14); and a mouthpiece (2) that holds the electrode (14) and the potential controller and is attached to the dental implant (8). The peri-implantitis treatment device (1) according to the present invention makes it possible to effectively inactivate even bacteria (colonies) that have formed biofilms hard to inactivate by antibiotics in deep periodontal pockets where mechanical cleaning is difficult.

## Description

### Technical Field

The present invention relates to a peri-implantitis treatment device and a biofilm activity adjusting method.

### Background Art

It is known that a probing pocket depth (PPD) is deepened by invasion of bacteria into a groove between a dental implant and a gingiva, causing a condition called a peri-implantitis.

When a peri-implantitis develops, e.g. mechanical cleaning using a device described in PTL 1, cleaning with an antimicrobial agent, and a local or systemic drug therapy with an antimicrobial agent, and the like are sequentially tried, depending on a depth of PPD.

### Citation List

### Patent Literature

PTL 1: JP 2017-532175 W

### Summary of Invention

### Technical Problem

Even when a mechanical cleaning and a treatment with an antimicrobial agent are sequentially tried because of development of peri-implantitis, an alveolar bone is eventually lost due to bacterial infection, which often forces surgical removal of the dental implant.

Generally, there are two reasons why surgical removal of implants is selected.

One reason why is because, mechanical cleaning becomes more difficult as the PPD deepens, although mechanical cleaning of a deep periodontal pocket is considered to be effective for preventing peri-implantitis and suppressing its progression. In other words, the deeper the PPD is, the more difficult the removal of the causative bacterial colonies around the implant is. Thus, treatment becomes more difficult, which eventually forces removal of the implant.

The other reason why is because the causative bacterial colonies form biofilms. Since washing, cleaning, and sterilization using antimicrobial agents hardly exhibit effects on the causative bacterial colonies that have formed the biofilms, mechanical cleaning as well as drug therapy and the like are insufficiently effective.

Considering the characteristic of peri-implantitis that becomes more difficult to treat due to the property of the PPD that gradually deepens, an object of the present invention is to provide a peri-implantitis treatment device capable of effectively inactivating even bacteria (colonies) that have formed biofilms hard to inactivate by antibiotics in deep periodontal pockets where mechanical cleaning is difficult.

Another object of the present invention is to provide a biofilm activity adjusting method applicable for studying bacteria (colonies) that have formed biofilms, and the like.

### Solution to Problem

As a result of intensive studies for solving the above problems, the present inventors have found that the above problems can be solved by the following configurations.

[1] A peri-implantitis treatment device including: an electrode for applying a negative electric potential to a metal portion of a dental implant; a potential controller for controlling the electric potential of the electrode; and a mouthpiece that holds the electrode and the potential controller and is attached to the dental implant.
[2] The peri-implantitis treatment device according to [1], in which the potential controller controls the electric potential of the electrode to a value of higher than a lower limit value of an electric potential window and lower than -0.4 V with respect to a silver/silver chloride electrode reference.
[3] The peri-implantitis treatment device according to [1] or [2], in which the electrode has a current density of lower than 1.0 µA/cm².
[4] A biofilm activity adjusting method, including applying, to a conductor having biofilms, an electric potential of higher than a lower limit value of an electric potential window and lower than -0.4 V with respect to a silver/silver chloride electrode reference to inactivate the biofilms.
[5] The biofilm activity adjusting method according to [4], including: bringing the conductor into direct contact with a bacterial suspension; and applying a positive electric potential to the conductor to form biofilms.
[6] The biofilm activity adjusting method according to [4] or [5], in which the inactivation is performed in a liquid medium, the method further includes: comparing optical density (OD) values of the liquid medium between before and after the inactivation; and providing information for determining a degree of progression of the inactivation depending on an increase in the OD value.
[7] The biofilm activity adjusting method according to any one of [4] to [6], in which the conductor has a current density of lower than 1.0 µA/cm².
[8] The biofilm activity adjusting method according to any one of [4] to [7], in which the biofilms are formed by oral bacteria.

### Advantageous Effects of Invention

The present invention makes it possible to provide a peri-implantitis treatment device capable of effectively inactivating even bacteria (colonies) that have formed biofilms hard to inactivate by antibiotics in deep periodontal pockets where mechanical cleaning is difficult.

Also, the present invention makes it possible to provide a biofilm activity adjusting method applicable for studying bacteria (colonies) that have formed biofilms, and the like.

The peri-implantitis treatment device according to the present invention (hereinafter, also referred to as "the present device") includes an electrode for applying a negative electric potential to a metal portion of a dental implant; a potential controller for controlling the electric potential of the electrode; and a mouthpiece that holds the electrode and the potential controller and is attached to the dental implant.

The present device has an electrode for applying a negative electric potential to the metal portion of the dental implant. According to the inventor's study, it is assumed that causative bacterial colonies of peri-implantitis adhere to a metal portion of a dental implant to form biofilms in periodontal pockets of a dental implant, and exhibit pathogenicity under anaerobic environment in the biofilms, as described in Examples below.

In this case, the causative bacterial colonies are assumed to use the metal portion of the dental implant as an acceptor for electrons released accompanying metabolism of electron sources (e.g., organic compounds) (electron acceptor).

In the device according to the present invention, a negative electric potential is applied to a metal portion of a dental implant to suppress electron transfer to the metal portion and metabolism of causative bacteria (colonies), so that their activity can be decreased (inactivated).

Furthermore, since the electron transfer occurs between the biofilm in direct contact with the metal portion of the dental implant and the metal portion, its effect is more direct and extremely more advantageous compared to drug therapies using antimicrobial agents whose effect is reduced by the biofilm.

When the potential controller of the present device controls the electric potential of the electrode to a value higher than a lower limit value of an electric potential window and lower than -0.4 (vs. Ag/AgCl) V, a superior inactivation effect is obtained. Primarily, if the electric potential of the electrode (i.e. electric potential of the metal portion of the dental implant) is controlled to an electric potential higher than the lower limit value of the electric potential window, hydrogen generation is more suppressed. Thereby, electrons (e⁻) more readily transfer from the electrode to the causative bacteria (colonies), and the inactivation effect is improved.

Secondly, if the electric potential of the electrode is controlled to a value lower than -0.4 (vs. Ag/AgCl) V, the causative bacteria (colonies) hardly transfer electrons to the electrode, and therefore the metabolism of the electron sources (organic compounds, etc.) is suppressed, resulting in a superior inactivation effect.

If the current density is lower than 0.1 µA/cm², even when the device is attached to a living body, a burden on the living body can be easily reduced. The lower limit value of the current density is preferably, but not particularly limited to, 0.1 nA/cm² or higher from the viewpoint of obtaining a superior inactivation effect.

The biofilm activity adjusting method according to the present invention (hereinafter, also referred to as "the present method") includes applying, to a conductor having biofilms, an electric potential of higher than a lower limit value of an electric potential window and lower than -0.4 (vs. Ag/AgCl) V to inactivate the biofilms.

When an electric potential of higher than the lower limit value of the electric potential window is applied to a conductor having biofilms, hydrogen generation can be more suppressed. This facilitates the transfer of electrons (e⁻) from the conductor to the causative bacteria (colonies), so that an inactivation effect on the biofilms is obtained.

Application of an electric potential of lower than -0.4 (vs. Ag/AgCl) V to the conductor makes it more difficult for the bacteria (colonies) to transfer electrons to the conductor, resulting in a superior activity adjustment effect.

When the present method includes bringing the conductor into direct contact with a bacterial suspension and applying a positive electric potential to the conductor to form biofilms, the method can be utilized for studying formation and disappearance of biofilms by certain bacteria, agents that promote or inhibit the formation or disappearance, and the like.

If the inactivation by the method is performed in a liquid medium and the method further includes comparing the OD values of the liquid medium between before and after the inactivation and providing information for determining a degree of progression of the inactivation depending on an increase in the OD value, progression of the inactivation can be more easily managed without checking the state of the biofilms through microscopic observation, or the like.

If the current density in the present method is lower than 0.1 µA/cm², influences on a living body can be reduced when the conductor is an implant or the like that is placed in the living body. The lower limit value of the current density is preferably, but not particularly limited to, 0.1 nA/cm² or higher from the viewpoint of obtaining a superior inactivation effect.

When the biofilms in the present method are formed by oral bacteria, the present method can be easily applied to development of peri-implantitis preventing methods, or the like.

### Brief Description of Drawings

[Fig. 1] Fig. 1 is a perspective view illustrating an example of a peri-implantitis treatment device according to the embodiment of the present invention.
[Fig. 2] Fig. 2 is a schematic sectional view of a peri-implantitis treatment device 1 attached to a mandibular first premolar.
[Fig. 3] Fig. 3 is a hardware configuration diagram of the peri-implantitis treatment device 1.
[Fig. 4] Fig. 4 is a functional block diagram of the peri-implantitis treatment device 1.
[Fig. 5] Fig. 5 is a flow chart according to the embodiment of the present method.
[Fig. 6] Fig. 6 is a scanning electron microscope image of a biofilm formed on an electrode.
[Fig. 7] Fig. 7 is a partially-enlarged scanning electron microscope image of the biofilm formed on the electrode.
[Fig. 8] Fig. 8 is a result of a current value measurement.
[Fig. 9] Fig. 9 is a scanning electron microscope image of an electrode surface after 15 hours from start of potential application (-400 mV).
[Fig. 10] Fig. 10 is a partially-enlarged image of Fig. 9.
[Fig. 11] Fig. 11 is a scanning electron microscope image of the electrode surface after 15 hours from start of potential application (-600 mV).
[Fig. 12] Fig. 12 is a partially-enlarged image of Fig. 11.
[Fig. 13] Fig. 13 is a scanning electron microscope image of the electrode surface after 15 hours from start of potential application (-800 mV).
[Fig. 14] Fig. 14 is a partially-enlarged image of Fig. 13.

### Description of Embodiments

The present invention will be explained below in detail.

Although the explanation of the constituent elements described below is based on representative embodiments of the present invention in some cases, the present invention is not limited to such embodiments.

In this specification, a numerical value range expressed by "to" means a range including the numerical values described before and after "to" as lower and upper limit values.

### [Peri-Implantitis Treatment Device]

The peri-implantitis treatment device according to the embodiment of the present invention will be explained with reference to the drawings.

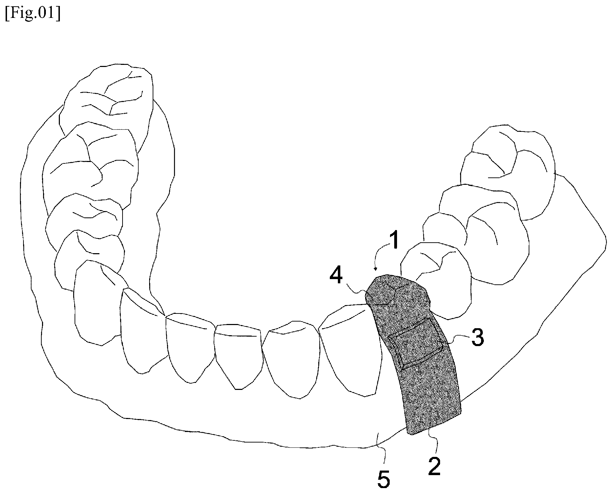

Fig. 1 is a perspective view illustrating an example of a peri-implantitis treatment device according to the embodiment of the present invention. In Fig. 1, the peri-implantitis treatment device 1 is composed of a band-shaped mouthpiece 2 that covers from a tooth crown portion of an implant-prosthetic first premolar 4 on a left lower jaw to a periodontal tissue 5, and a substrate accommodating portion 3 that accommodates a substrate having each hardware described below.

Although the peri-implantitis treatment device 1 has a shape that covers one prosthetic tooth, the shape of the peri-implantitis treatment device according to the present invention is not limited to the shape of the device 1, and may be a shape that also cover a plurality of prosthetic teeth or a plurality of teeth other than prosthetic teeth (i.e. healthy teeth) together. The device 1 may also have a shape that covers all teeth on the lower or upper jaw.

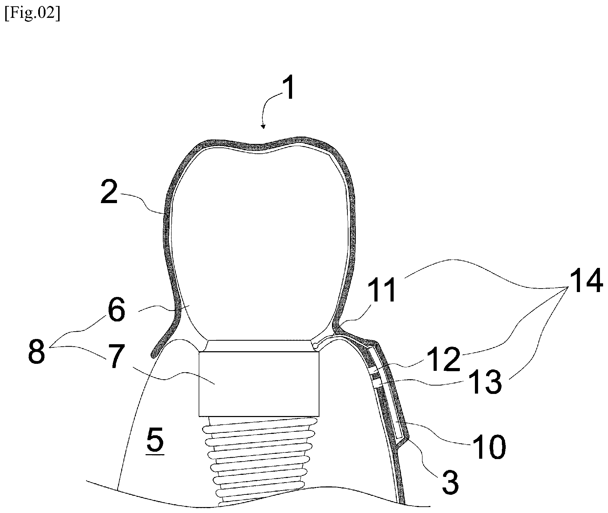

Fig. 2 is a schematic sectional view of the peri-implantitis treatment device 1 attached to a mandibular first premolar. The peri-implantitis treatment device 1 has: the mouthpiece 2 arranged so as to cover a dental implant 8 composed of a tooth crown portion 6 and a metal portion 7 including an implant body and an abutment; and the substrate accommodating portion 3 located on the mouthpiece 2.

A substrate 10 on which each hardware described below is mounted is disposed in the substrate accommodating portion 3. The substrate 10 is connected with an electrode 14 composed of a working electrode 11, a counter electrode 12, and a reference electrode 13. The working electrode 11 is in direct contact with the metal portion 7 of the dental implant 8, and the counter electrode 12 and the reference electrode 13 are in direct contact with the periodontal tissue 5.

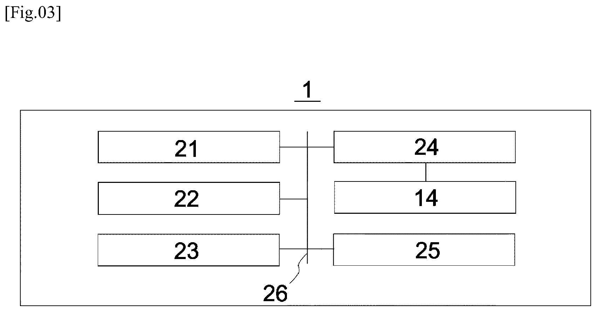

Fig. 3 is a hardware configuration diagram of the peri-implantitis treatment device 1. The peri-implantitis treatment device 1 includes a processor 21, a memory device 22, a communication interface (I/F) 23, a potential controller 24 connected with the electrode 14, and a power source 25. The respective constituents are communicatively connected to each other via a bus 26 and mounted on the substrate 10.

The processor 21 controls each constituent of the peri-implantitis treatment device 1 to effect a function.

Examples of the processor 21 include microprocessors, processor cores, multiprocessors, application-specific integrated circuits (ASIC), field programmable gate arrays (FPGA), and general-purpose computing on graphics processing units (GPGPU).

The memory device 22 temporarily and/or non-temporarily stores various programs and data and provides a work area for the processor 21.

Examples of the memory device 22 include read only memories (ROM), random access memories (RAM), hard disk drives (HDD), flash memories, and solid state drives (SSD).

The potential controller 24 controls the electric potential of the electrode 14 and measures the current value. Specifically, a negative electric potential is applied to the metal portion 7 of the dental implant 8 to measure the current value. The potential controller 24 is typically a potentiostat and is built into the substrate 10.

The electrode 14 is composed of a working electrode 11 for applying a negative electric potential to the metal portion 7 of the dental implant 8, as well as a counter electrode 12 and a reference electrode 13.

In the electrode 14, the working electrode 11 is in direct contact with the metal portion 7 of the dental implant 8. Generally, the metal portion 7 of the dental implant 8 is composed of a tooth root portion (implant body) and a support base portion (abutment), and is made of a titanium alloy, a pure titanium, a Co-Cr-Mo alloy, or the like. On the other hand, the counter electrode 12 and the reference electrode 13 are in contact with the periodontal tissue 5.

Materials of the working electrode 11 and the counter electrode 12 are not particularly limited, and any known electrode materials may be used. The reference electrode 13 is a silver/silver chloride electrode, but another known reference electrode may be used.

The working electrode 11 must be in contact with the metal portion 7 of the dental implant 8, but the counter electrode 12 and the reference electrode 13 only need to be in contact with any part of the oral cavity, and may have not only a shape in contact with the periodontal tissue 5 as illustrated in Fig. 2 but also a shape in contact with an inner mucosa of cheek, a mucosa under tongue, a mucosa between upper gums on both sides, and the like.

The power source 25 supplies an electric power necessary for driving each constituent of the peri-implantitis treatment device 1. The power source 25 is typically an all-solid battery and surface-mounted on the substrate 10. The all-solid battery can be charged e.g. by a wireless charging system described in JP 2020-191772 A, or the like.

The power source 25 is not limited to the all-solid battery, and another conventionally known battery (e.g., lithium-ion batteries) or the like may be used. The power source 25 need not be surface-mounted on the substrate 10.

The communication interface (I/F) 23 executes communication for inputting, from the outside, operating conditions and the like of the potential controller 24, and for outputting, to the outside, an electric potential of the working electrode 11 controlled by the potential controller 24, a current value, and the like measured by the potential controller 24.

Although the peri-implantitis treatment device 1 has the processor 21, the memory device 22, the communication interface 23, and the potential controller 24, the peri-implantitis treatment device according to the present invention is not limited to the above configuration and may have a microcomputer that has some or all functions of the constituents described above.

The mouthpiece 2 holds the substrate 10 including the constituents described above in the substrate accommodating portion 3 and is attached to the tooth crown portion 6 while the working electrode 11 is in direct contact with the metal portion 7.

A void in the substrate accommodating portion 3 may be sealed by a sealing material.

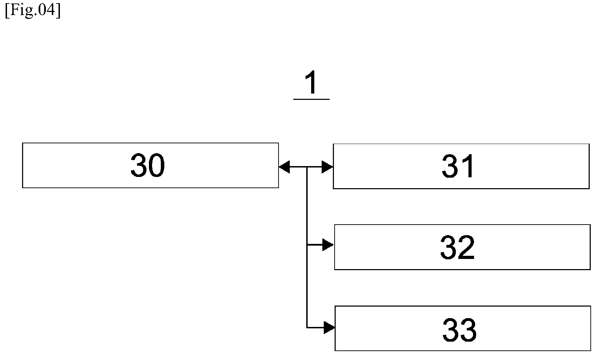

Fig. 4 is a functional block diagram of the peri-implantitis treatment device 1. The peri-implantitis treatment device 1 is composed of a control portion 30, a communication portion 31, a potential controlling portion 32, and a memory portion 33.

The control portion 30 includes a processor 21 and controls each of the communication portion 31, the potential controlling portion 32, and the memory portion 33 to effect the function of the peri-implantitis treatment device 1.

The memory portion 33 includes the memory device 22. The memory portion 33 has functions of reading out programs required for controlling each constituent as necessary, storing information (electric potentials and current values) acquired by the potential controller 24, and reading out these information according to requests of the control portion 30.

The communication portion 31 includes the communication interface 23. The function of the communication portion 31 is effected when programs stored in the memory device 22 are executed by the processor 21 and the communication interface 23 is controlled.

The communication portion 31 has functions of receiving an external input of operating conditions (e.g., an electric potential to be applied, and a duration of potential application) of the potential controller 24; transmitting data of a current value acquired by the potential controller 24 to the outside; and transmitting logs and the like regarding the operation of the device to the outside.

The potential controlling portion 32 includes the potential controller 24 and the electrode 14 connected thereto. The function of the potential controlling portion 32 is effected when programs stored in the memory device 22 are executed by the processor 21 and the potential controller 24 is controlled.

The potential controlling portion 32 has a function of applying a negative electric potential to the metal portion 7 via the working electrode 11 in contact with the metal portion 7 of the dental implant 8 to measure a current value.

When treating peri-implantitis using the peri-implantitis treatment device 1, first, the mouthpiece 2 is attached to the dental implant 8 such that the working electrode 11 comes into contact with the metal portion 7 of the dental implant 8 to be treated.

Next, the electric potential of the electrode 14 is controlled by the potential controlling portion 32 according to conditions previously stored in the memory portion 33 or conditions input from the outside via the communication portion 31 to apply a negative electric potential to the metal portion 7 of the dental implant 8.

The applied electric potential is preferably higher than a lower limit value of an electric potential window and lower than -0.4 (vs. Ag/AgCl) V A duration of potential application depends on the progression of peri-implantitis or the like, and may be adjusted as appropriate. Generally, the duration is preferably 1 to 24 hours.

For example, a person wears the peri-implantitis treatment device 1 at bedtime, and the device is operated for about 5 to 10 hours, and thereby the biofilms in periodontal pockets can be sufficiently inactivated. This process is repeated to suppress the progression of the peri-implantitis and treat the peri-implantitis.

### [Biofilm Activity Adjusting Method]

Next, the method according to the present invention will be explained. The method according to the present invention is a biofilm activity adjusting method, including applying, to a conductor having biofilms, an electric potential of higher than a lower limit value of an electric potential window and lower than -0.4 (vs. Ag/AgCl) V to inactivate the biofilms.

A conductor having biofilms means e.g. a medical appliance placed in a living body such as a dental implant, an examination appliance such as an endoscope, and a metal portion of a medical appliance for use in a surgical operation.

If the method is applied to such a conductor, the biofilms can be inactivated over small parts having complicated shapes, where mechanical cleaning is inaccessible. Thereby, bacterial infection can be suppressed, and the conductor can be used in a cleaner state.

The conductor having the biofilms may be an electrode or the like for use in a laboratory. For example, if biofilms formed by culturing microorganisms on a transparent electrode are inactivated according to the present method, the electrode can be applied for observing the biofilm morphology during the inactivation process, or for studying utilization of the electrode for changes in an electric current obtained in the inactivation process, or the like.

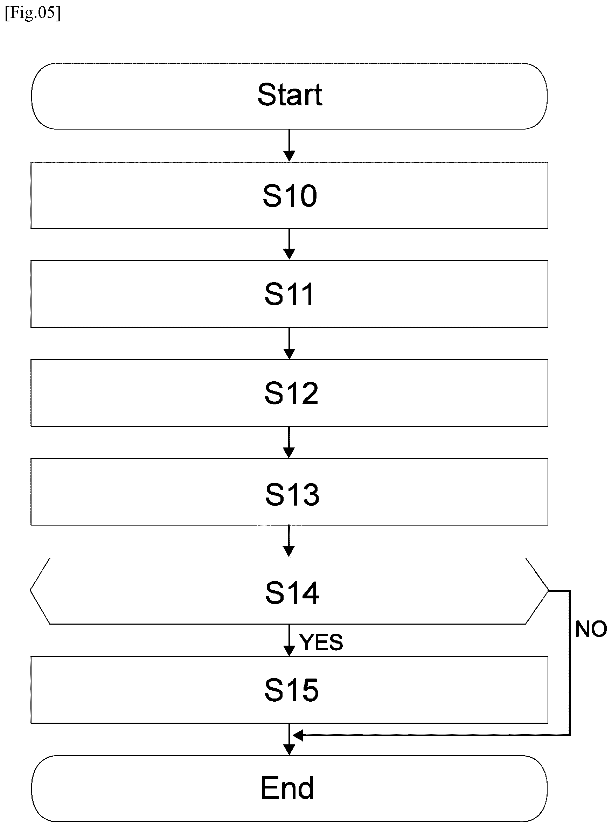

Fig. 5 is a flow chart according to the embodiment of the present method.

First, a positive electric potential is applied to a conductor in a liquid medium to form biofilms on the conductor (step S10).

The liquid medium contains bacterial colonies capable of forming biofilms, and water, and may additionally contain electrolytes, electron sources (organic compounds), and the like.

The electric potential is preferably, but not particularly limited to, an electric potential of lower than an upper limit value of an electric potential window.

In the method according to the embodiment of the present invention, a conductor on which biofilms have been already formed may be used, and in this case, step S10 can be omitted.

Subsequently, an OD (optical density) value of the liquid medium is measured (step S11). The OD value correlates with the amount of suspended bacterial colonies in the liquid medium. After the biofilms are formed, the liquid medium may be replaced with a new liquid medium. When the liquid medium is replaced after the biofilms are formed, it is preferable that the liquid medium to be replaced contains no bacteria. Use of a liquid medium containing no bacteria makes it possible to obtain more accurate information for evaluating the progression of the biofilm inactivation when a negative electric potential is applied to the conductor.

Then, an electric potential of higher than the lower limit value of the electric potential window and lower than -0.4 (vs. Ag/AgCl) V is applied to the conductor (step S12). The method for applying the electric potential is not particularly limited, and for example, it is possible to adopt a method in which a conductor having biofilms is used as a working electrode, furthermore a counter electrode and a reference electrode are brought into contact with a liquid medium, and these electrodes are controlled by a potentiostat.

A duration of potential application is not particularly limited and should be selected depending on an intended purpose. As an example, the duration is 1 to 24 hours.

Next, the OD value of the liquid medium is measured (step S13). When the biofilms are inactivated by applying an electric potential, the bacterial colonies and the like in the biofilms are suspended in the liquid medium. Thus, the OD value changes between before and after the application of the negative electric potential. This change in the OD value can be used as an indicator of inactivation.

If the OD value increases by applying the electric potential (step S14: YES), there is a high probability that bacteria have been released into the liquid medium because of progression of the inactivation. In this case, information for determining progression of the inactivation is provided (Step S15).

Examples of this information include a difference (increase) in OD values between before and after application of the negative electric potential, an OD value after application of the negative electric potential, and an increase in the OD value per a unit time.

### Examples

Results of an experiment for demonstrating a mechanism of peri-implantitis treatment using the device will be explained below. Note that the following experimental data are only for the illustrative purposes, and are not intended to limit the operating conditions and the like of the present device.

The experiment was performed using a three-electrode electrochemical cell having an indium tin oxide (ITO) electrode as the working electrode, a platinum electrode as the counter electrode, and a silver/silver chloride electrode as the reference electrode, under anaerobic condition caused by nitrogen bubbling.

First, a liquid culture medium containing 10 mM of lactate (defined media) as an electron source was added to the cell, to which Streptococcus mutans (OD: 0.1) was inoculated, which was incubated with a working electrode potential of +0.2 V (200 mV, vs. Ag/AgCl) at 37°C for 24 hours to form a biofilm.

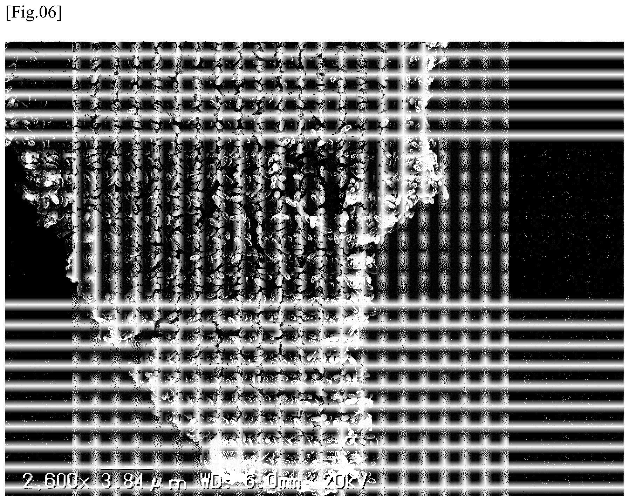

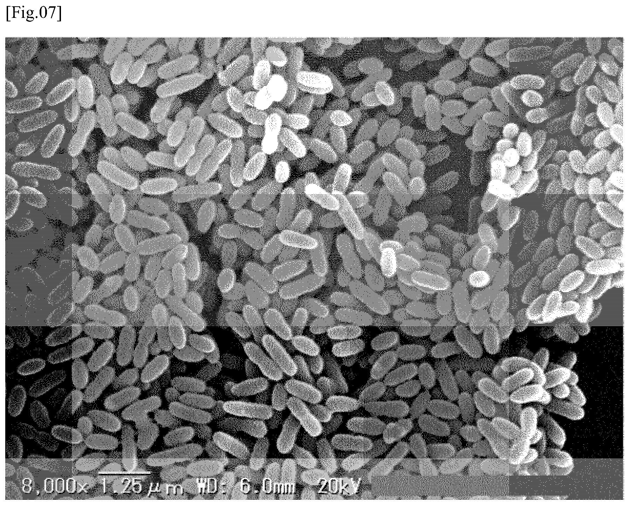

Fig. 6 is a scanning electron microscope image of a biofilm formed on an electrode, and Fig. 7 is a partially-enlarged image of the biofilm. Both figures show thick bacterial aggregates (biofilm) formed on the electrode.

Next, the liquid medium was once removed, and a new liquid medium (sterile) was added to the cell, and, at this time, an OD value (562 nm) was measured.

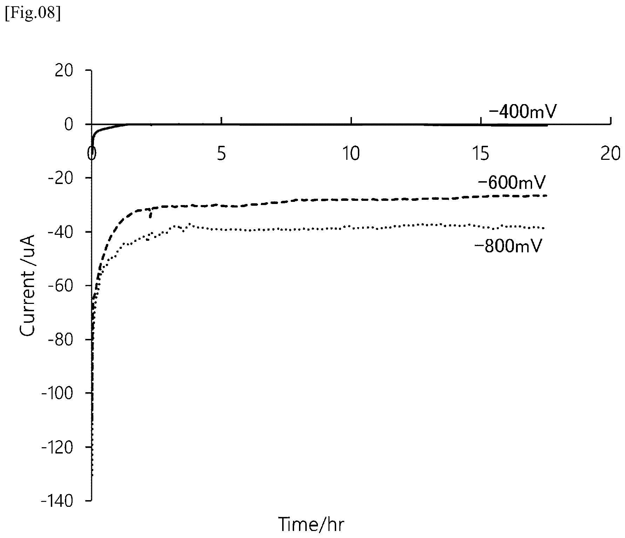

Subsequently, the electric potential of the working electrode was controlled to -400 mV, -600 mV, and -800 mV, the current values were measured, and OD values were measured after 2 hours and 16 hours from the start of the potential application. Fig. 8 presents a result of the current value measurement, and Table 1 presents a result of the OD measurement. The lower limit value of the electric potential window in this experimental system has been experimentally confirmed to be about -1.0 V (vs. Ag/AgCl), and the value -800 mV is higher than the lower limit value of the electric potential window.

**[Table 1]**

| Table 1 | OD value (time from start of potential application) | | |
|---|---|---|---|
| Potential (mV) | 0 hour | 2 hours | 16 hours |
| -400 | 0.022 | 0.039 | 0.252 |
| -600 | 0.021 | 0.158 | 0.159 |
| -800 | 0.01 | 0.128 | 0.221 |

The result in Table 1 showed that, at all potentials, the OD values after the elapse of 2 hours increased compared to those at the start of the potential application, and after 16 hours, the OD values sufficiently increased.

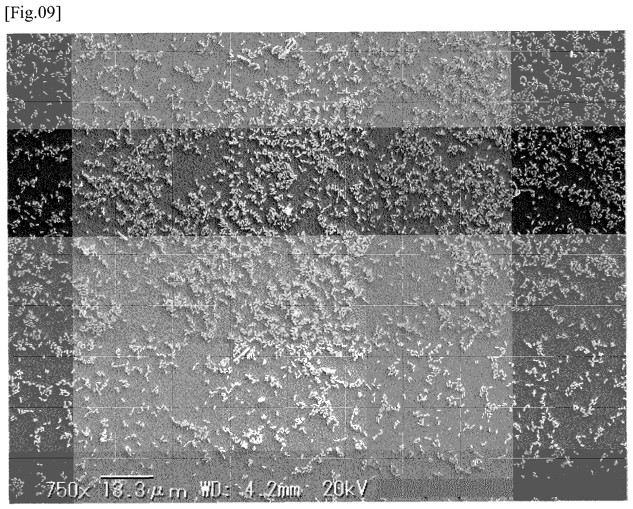

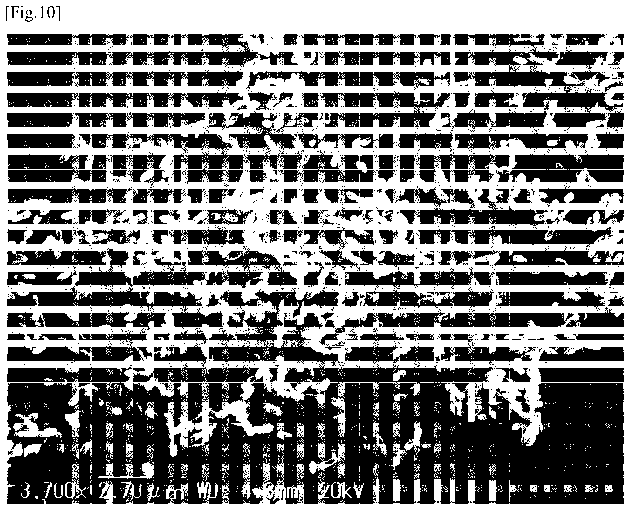

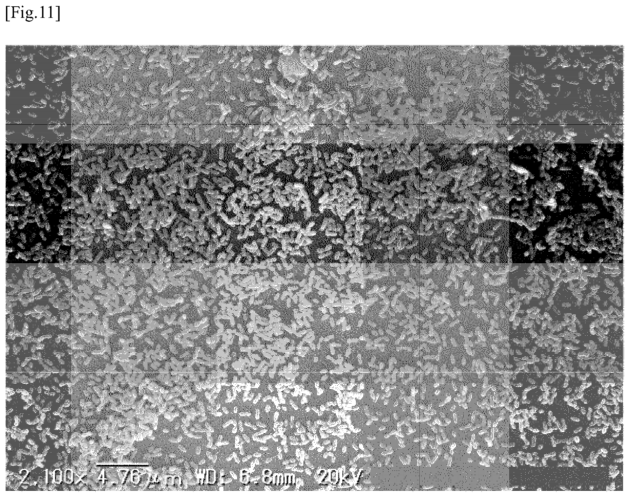

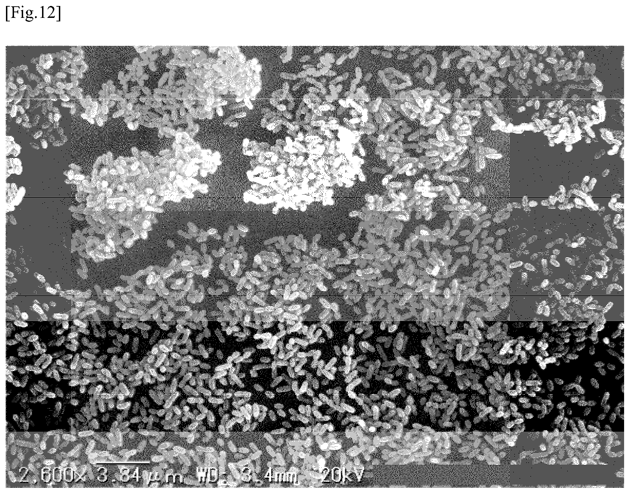

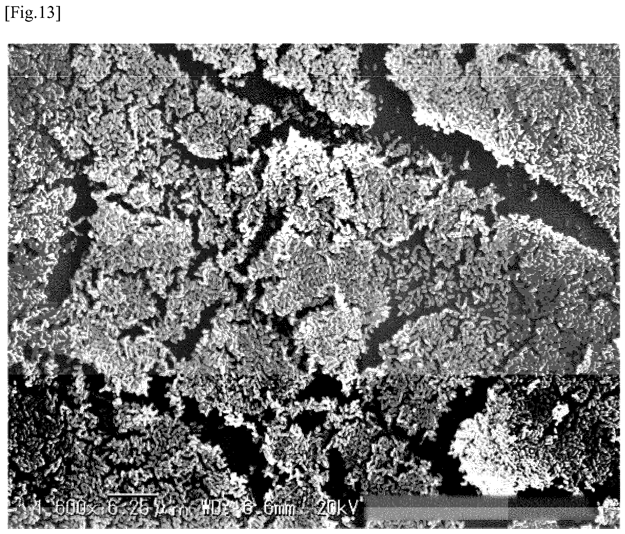

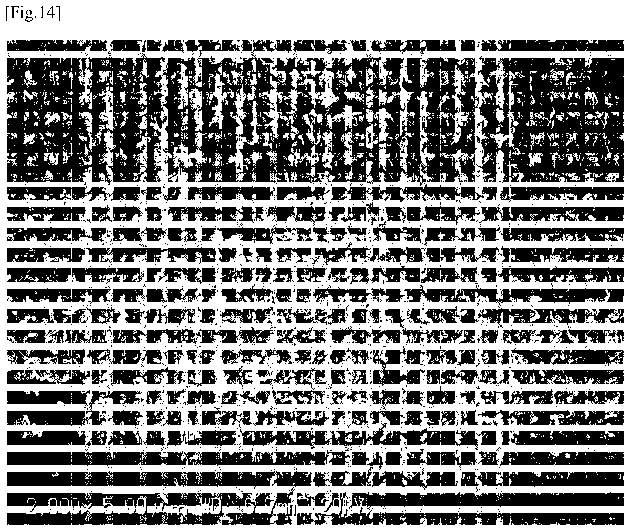

Fig. 9 to Fig. 14 are scanning electron microscope images of the electrode surfaces after 15 hours from the start of the potential application. Fig. 9 is an image at -400 mV, and Fig. 10 is a partially-enlarged image of Fig. 9. Fig. 11 is an image at -600 mV, and Fig. 12 is a partially-enlarged image of Fig. 11. Fig. 13 is an image at -800 mV, and Fig. 14 is a partially-enlarged image of Fig. 13.

In all cases of Fig. 9 to Fig. 14, it was confirmed that the bacterial aggregates on the electrode had been destroyed, i.e. the biofilms had been inactivated compared to Fig. 6.

### Industrial Applicability

The peri-implantitis treatment device according to the present invention makes it possible to effectively inactivate even bacterial colonies that have formed biofilms hard to inactivate by antibiotics in deep periodontal pockets where mechanical cleaning is difficult.

The peri-implantitis treatment device according to the present invention is thought to be effective also for prevention of peri-implantitis, and if a dental implant user uses the device on a regular basis, medical expenses can be reduced.

## Claims

1. A peri-implantitis treatment device comprising:
an electrode for applying a negative electric potential to a metal portion of a dental implant;
a potential controller for controlling the electric potential of the electrode; and
a mouthpiece that holds the electrode and the potential controller and is attached to the dental implant.

2. The peri-implantitis treatment device according to claim 1, wherein the potential controller controls the electric potential of the electrode to a value of higher than a lower limit value of an electric potential window and lower than -0.4 V with respect to a silver/silver chloride electrode reference.

3. The peri-implantitis treatment device according to claim 1 or 2, wherein the electrode has a current density of lower than 1.0 µA/cm².

4. A biofilm activity adjusting method, comprising applying, to a conductor having biofilms, an electric potential of higher than a lower limit value of an electric potential window and lower than -0.4 V with respect to a silver/silver chloride electrode reference to inactivate the biofilms.

5. The biofilm activity adjusting method according to claim 4, comprising: bringing the conductor into direct contact with a bacterial suspension; and applying a positive electric potential to the conductor to form biofilms.

6. The biofilm activity adjusting method according to claim 4 or 5, wherein
the inactivation is performed in a liquid medium, and
the method further comprises: comparing optical density (OD) values of the liquid medium between before and after the inactivation; and providing information for determining a degree of progression of the inactivation depending on an increase in the OD value.

7. The biofilm activity adjusting method according to any one of claims 4 to 6, wherein the conductor has a current density of lower than 1.0 µA/cm².

8. The biofilm activity adjusting method according to any one of claims 4 to 7, wherein the biofilms are formed by oral bacteria.
